# EUROPÄISCHE PATENTSCHRIFT

(11) **EP 1 728 489 B1**
(45) Veröffentlichungstag und Bekanntmachung des Hinweises auf die Patenterteilung: **19.07.2017**
(21) Anmeldenummer: 06008525.5
(22) Anmeldetag: 25.04.2006
(51) Int. Cl.: A61F 2/32, A61F 2/36, A61B 17/86, A61F 2/30, A61F 2/34, A61F 2/46

(54) **Hüftgelenk-Endoprothese mit Gelenkpfanne mit abgeschlossenen Bohrungen**
Hip endoprosthesis with acetabular having sealed bores
Prothèse d'articulation de la hanche ayant de cupule cotyloidienne avec des trous fermés

(30) Priorität: 03.06.2005 DE 102005025686
(43) Veröffentlichungstag der Anmeldung: 06.12.2006
(73) Patentinhaber: Mathys AG Bettlach, 2544 Bettlach (CH)
(72) Erfinder: Delfosse, Daniel, Dr., 3306 Jegensdorf (CH); Cotting, Anton, 3540 Grenchen (CH)
(74) Vertreter: Körfer, Thomas

(56) Entgegenhaltungen:
- EP-A- 0 346 294
- EP-A- 0 499 475
- EP-A- 1 338 256
- WO-A-03/039410
- WO-A1-95/15734
- FR-A- 2 838 329
- US-A- 5 370 702
- US-A- 5 645 606
- US-A- 5 888 205
- US-A- 5 925 077
- US-A1- 2003 135 281

## Beschreibung

Die Erfindung betrifft eine Hüftgelenk-Endoprothese zur prothetischen Versorgung des menschlichen oder tierischen Hüftgelenks mit einer abgeschlossenen Gelenkpfanne.
Einteilige Hüftgelenkspfannen aus Kunststoff werden gewöhnlich zementfrei durch Einspreizen in einer vorbereiteten Knochenkavität verankert. Als zusätzliche Sicherung sind Schrauben vorgesehen, welche durch dafür vorgesehene Bohrungen in der Hüftgelenkspfanne bei Bedarf in den Knochen eingeschraubt werden können.

Nachteilig an diesen bekannten Hüftgelenkspfannen ist insbesondere, daß bei Nichtverwendung der Bohrungen zur Fixierung der Hüftgelenkspfanne diese offen bleiben und somit die Gefahr des Einwanderns von Knochenabrieb und Knochenspänen von der vorbereitenden Bearbeitung des Beckenknochens in die Hüftgelenkspfanne einwandern und zwischen dieser und der Gelenkkugel zu verstärkter Reibung und somit zu Abnutzung führen.
Aus vielen Krankheitsbildern und Publikationen ist auch der umgekehrte Partikelfluß bekannt. Die feinen Partikel können durch die Bohrungen aus der Artikulationszone direkt ins Acetabulum wandern und dort die gefürchtete Osteolyse auslösen, die letztlich zu einer Pfannenlockerung führen kann.
Die US-A-5 925 077 beschreibt eine Vorrichtung und ein Verfahren, um Löcher in einer Hüftgelenkpfannenkomponente zu verstopfen. Die Hüftgelenkpfannenkomponente weist eine innere konkave Oberfläche und eine äußere konvexe Oberfläche mit zumindest einem Loch, welches sich dadurch hindurch erstreckt, auf. Das Loch wird durch eine zylindrische mit einem Gewinde versehene Seitenwand definiert. Die Vorrichtung weist weiterhin einen Stöpsel aus Polymethylmethacrylat auf, welcher eine konische mit Gewinde versehen Seitenwand aufweist. Die zylindrische mit Gewinde versehene Seitenwand des Lochs nimmt die konische mit Gewinde versehene Seitenwand des Stöpsels auf, um eine Presspassung vorzusehen. Die Presspassung dichtet das Loch im Wesentlichen ab, um eine Migration von Abrieb durch das Loch hindurch zu verhindern.

In der FR-A-2 838 329 wird eine Gelenkpfannenstruktur beschrieben, welche aus einem pfannenartigen Körper mit einem oder mehreren Löchern zu dessen Fixierung mittels Schrauben im Beckenknochen aufweist. Jedes Loch ist mit einem zylindrischen Stöpsel versehen, welcher als zusätzliche Komponente provisorisch in das Loch eingesetzt ist und bei Durchbohrung des Loches mit einer Schraube herausgedrückt wird.

In der US-A-5 888 205 wird eine Hüftgelenkpfanne beschrieben, welche ebenfalls mittels Schrauben, welche durch in der Pfanne vorgesehene Löcher hindurchfühbar sind, in dem Beckenknochen eines Patienten fixiert werden kann.

Die EP-A-1 338 256 beschreibt eine Hüftgelenkpfannenkomponente, wobei die Pfanne Bereiche mit verringerter Dicke aufweist, welche aus durch Ausstanzen mit einem speziellen Werkzeug Löcher bilden, durch welche Schrauben hindurchführbar sind, um die Hüftpfannenkomponente in einem Beckenknochen eines Patienten zu fixieren.

In der US 2003/135281 A1 wird eine zweischalige Hüftgelenkpfanne beschrieben, welche aus einer äußeren Wand und einer inneren Wand besteht. Die äußere Wand definiert eine äußere Oberfläche der Pfanne und die innere Wand definiert dabei eine innere Oberfläche, welche einen Pfannenhohlraum umgibt. Die äußere Wand ist aus einem porösen Material hergestellt, in welches Knochenmaterial hineinwachsen kann. Löcher für Schrauben erstrecken sich durch die äußere und die innere Wand. Weiterhin sind Kanäle in der inneren Wand der Pfanne vorgesehen, welche teilweise durch die äußere Wand abgedeckt sein können.

In der EP-A-0 499 475 wird eine Hüftpfannenprothese beschrieben, welche einen pfannenartigen Körper mit einer Vielzahl von Bohrungen, welche sich durch eine innere konkave Oberfläche und eine äußere konvexe Oberfläche erstrecken, aufweist. Die Bohrungen dienen als Bohrungsführungen, um Löcher in das darunter liegende Gewebe zu bohren und einen durch die Bohrung hindurchführbaren Stift zur Fixierung aufzunehmen.

Der Erfindung liegt demnach die Aufgabe zugrunde, eine Hüftgelenk-Endoprothese mit einer Gelenkpfanne zu schaffen, welche so ausgebildet ist, daß die Gleitfläche der Gelenkpfanne frei von einwandernden Partikeln und umgekehrt das Acetabulum frei von Abriebpartikeln gehalten werden kann.

Die Aufgabe wird durch die kennzeichnenden Merkmale des Anspruchs 1 gelöst.
Gemäß Anspruch 1 ist vorgesehen, in den Bohrungen der Gelenkpfanne, welche der Aufnahme der Befestigungsschrauben dienen, eine Sperre in Form einer Membran einzubringen, so daß die Bohrungen bei Nichtverwendung nicht durchgängig sind und Knochenpartikel somit von der Gleitfläche ferngehalten werden können.

Weitere vorteilhafte Ausgestaltungen der Erfindung sind in den Unteransprüchen gekennzeichnet.
Ausführungsbeispiele der Erfindung werden im folgenden anhand teilweise schematischer Darstellungen in der Zeichnung näher erläutert. In der Zeichnung zeigen:
- Fig. 1: eine schematische Gesamtansicht eines Ausführungsbeispiels einer mit einer erfindungsgemäßen Gelenkpfanne ausgestattete Hüftgelenk-Endoprothese,
- Fig. 2A-B: schematische vergrößerte ausschnittsweise Darstellungen der erfindungsgemäß ausgestalteten Gelenkpfanne gemäß Fig. 1 im Bereich II in Fig. 1 vor und nach dem Durchdringen der Membran,
- Fig. 3: eine schematische vergrößerte Darstellung eines zweiten Ausführungsbeispiel einer erfindungsgemäß ausgestalteten Gelenkpfanne, und
- Fig. 4: eine schematische vergrößerte Darstellung eines dritten Ausführungsbeispiel einer erfindungsgemäß ausgestalteten Gelenkpfanne.

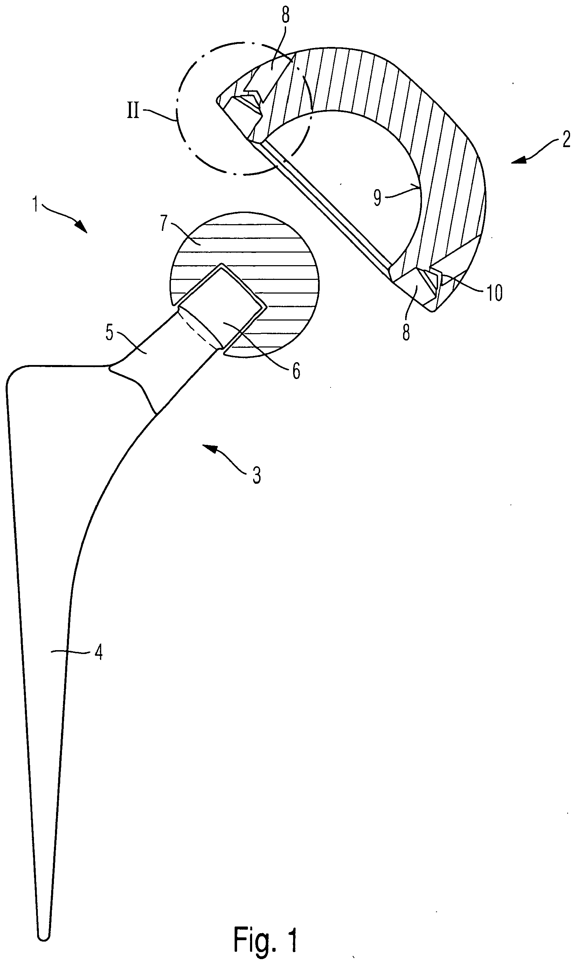

Fig. 1 zeigt in einer schematischen Darstellung eine Gesamtansicht einer Hüftgelenk-Endoprothese 1, die mit einer erfindungsgemäß ausgestalteten Gelenkpfanne 2 kombinierbar ist. Die im Ausführungsbeispiel dargestellte Hüftgelenk-Endoprothese 1 wird auch als totale Hüftgelenk-Endoprothese 1 bezeichnet, da sowohl der femurale als auch der beckenseitige Gelenkanteil ersetzt wird. Eine Femurkomponente 3 weist einen Schaft 4 auf, welcher in der Markhöhle eines Femurknochens je nach Bauform der Femurkomponente 3 einzementiert oder zementfrei verankert wird. An dem Schaft 4 ist eine Halspartie 5 ausgebildet, an deren Ende ein Hüftschaftkonus 6 zur Aufnahme einer Gelenkkugel 7 ausgebildet ist.

Die Femurkomponente 3 ist nur sehr schematisiert dargestellt und kann auch in beliebigen anderen Ausführungsformen ausgebildet sein, sofern eine Gelenkkugel 7 vorgesehen ist. Die im folgenden näher beschriebenen erfindungsgemäßen Maßnahmen sind somit unabhängig von der Form der Femurkomponente 3 zu sehen.
Die Gelenkkugel 7 liegt gleitend in einer Gelenkpfanne 2, welche aus Kunststoff, insbesondere aus vernetztem oder unvernetztem UHMWPE, einem Polymer, besteht und mittels im Ausführungsbeispiel nicht weiter dargestellter Schrauben im Beckenknochen verankerbar ist. Dies ist jedoch für die dargestellte Form der Gelenkpfanne 2 nicht zwingend nötig. Der Operateur kann auch entscheiden, die Gelenkpfanne 2 nur durch ihre Klemmwirkung im vorbereiteten Beckenknochen zu fixieren. Dann bleiben die für die Schrauben vorgesehenen Bohrungen 8 in der Gelenkpfanne 2 leer.
Nachteilig daran ist, daß durch die offenen Bohrungen 8 Knochenabrieb auf eine Gleitfläche 9 der Gelenkpfanne 2 bzw. umgekehrt Abriebpartikel in das Acetabulum gelangen können, was zu einem erhöhten Verschleiß, Osteolysen und zu vermehrt auftretenden Schadensfällen führen kann, was eine Revision der Hüftgelenk-Endoprothese 1 mit allen für den Patienten damit verbundenen Risiken bzw. den anfallenden Kosten notwendig macht.

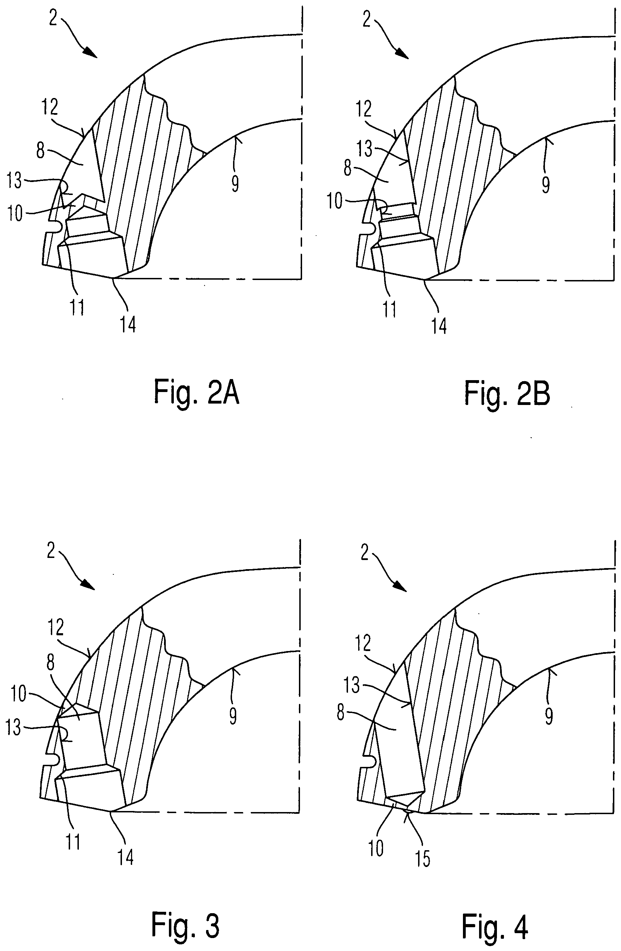

Erfindungsgemäß wird daher vorgeschlagen, während des Herstellungsprozesses oder anschließend an diesen in die Bohrungen 8 jeweils eine Sperre in Form einer Membran 10 einzubringen, welche die Bohrungen 8, wenn diese nicht zum Fixieren der Gelenkpfanne 2 verwendet werden, abschließen und so das Einwandern von Knochenabrieb auf die Gleitfläche 9 der Gelenkpfanne 2 verhindern bzw. umgekehrt feine Abriebpartikel durch die Membran gehindert werden, ins Acetabulum zu gelangen. Die erfindungsgemäß ausgestaltete Gelenkpfanne 2 wird im folgenden unter Bezugnahme auf die Fig. 2A und 2B näher beschrieben.
Fig. 2A zeigt dabei in stark schematisierter, vergrößerter Ansicht den in Fig. 1 mit II bezeichneten Ausschnitt aus der Gelenkpfanne 2. In der Bohrung 8 ist gut sichtbar die Membran 10 ausgebildet. Sie ist zwischen einer Schulter 11 der Bohrung 8, an welcher sich ein Kopf der nicht weiter dargestellten Befestigungsschraube abstützt, und einer beckenknochenseitigen Ausmündung 12 der Bohrung 8 angeordnet und im Querschnitt dreieckig, dreidimensional betrachtet also kegelförmig ausgebildet. Dies hat den Vorteil, daß bei Benutzung der Bohrungen 8 für die Schrauben die Spitze der Schraube zentriert wird und die Schraube die Membran 10 achsparallel zur der Bohrung 8 durchdringt. Ein Öffnungswinkel α der kegelförmigen Membran beträgt dabei vorzugsweise etwa 120°, was im Ausführungsbeispiel dem Öffnungswinkel im Bereich der Schulter 11 entspricht.
Fig. 2B zeigt in gleicher Darstellung wie Fig. 2A die Situation nach dem Einbringen einer Schraube in die Bohrung 8. Die Schraube ist aus Gründen der Übersichtlichkeit in Fig. 2B nicht dargestellt.
Bedingt dadurch, daß die Schraube die Membran 10 durchdringt, legt sich die Membran 10, wie aus Fig. 2B ersichtlich, nach dem Fixieren der Schraube kragenartig um diese herum, so daß auch bei eingebrachter Schraube eine Abdichtung der Bohrung 8 gegen Knochenabrieb gewährleistet ist.

Die Membran 10 kann dabei während des Herstellungsvorgangs der Gelenkpfanne 2 gleichzeitig mit dieser hergestellt werden, etwa, indem die Bohrung 8 mit geeigneten Bohrwerkzeugen in zwei Richtungen angebohrt, jedoch nicht vollständig durchgebohrt wird, bis nur noch die Membran 10 verbleibt.

Die Membran 10 kann weiterhin in ihrer axialen Position in der Bohrung 8 verschoben sein, indem sie beispielsweise, wie in Fig. 3 dargestellt, die knochenseitige Ausmündung 12 der Bohrung 8 abdeckt. Diese Ausführungsform ist vorteilhaft, da die Bohrung 8 dadurch nur von einer Seite in die Gelenkpfanne 2 eingebracht werden muß.
Auch eine Anordnung der Membran 10 als Abschluß einer gleitflächenseitigen Ausmündung 14 der Bohrung 8 ist möglich, wie in Fig. 4 dargestellt. Der Vorteil liegt in gleicher Weise wie in dem in Fig. 3 dargestellten Ausführungsbeispiel begründet, nämlich in der Tatsache, daß die Gelenkpfanne 2 nur von einer Seite angebohrt werden muß. Allerdings ist es bei dieser Ausführungsform notwendig, die Einschlagstelle der Schrauben mit einer Markierung 15 zu versehen, damit der Operateur die Position der in diesem Ausführungsbeispiel nach dem Implantieren der Gelenkpfanne 2 nicht mehr sichtbaren Bohrungen 8 auffinden kann. Die Markierung 15 kann beispielsweise in Form eines kleinen Überstands, wie in Fig. 4 dargestellt, oder in Form einer Farbmarkierung ausgeführt sein.
Die Erfindung ist nicht auf das dargestellte Ausführungsbeispiel beschränkt und beispielsweise auch für andere Formen von Femurkomponenten 3 anwendbar. Alle Merkmale der Erfindung sind beliebig miteinander kombinierbar.

## Patentansprüche

1. Hüftgelenk-Endoprothese (1), welche an einer Femurkomponente (3) eine Gelenkkugel (7) aufweist, die mit einer einteiligen Gelenkpfanne (2) aus Kunststoff eine Gleitpaarung bildet,
wobei die Gelenkpfanne (2) mit zumindest einer Schraube in einem Beckenknochen verankerbar ist und die zumindest eine Schraube in zumindest einer Bohrung (8) platzierbar ist und
wobei die zumindest eine Bohrung (8) durch eine Sperre abgeschlossen ist, wobei die Sperre eine Membran (10) ist,
wobei die Membran (10) einstückig mit der Gelenkpfanne (2) vorliegt, und
wobei die Membran (10) derart ausgebildet ist, dass die Schraube beim Einbringen in die Bohrung (8) die Membran (10) durchdringt.

2. Hüftgelenk-Endoprothese nach Anspruch 1,
**dadurch gekennzeichnet,**
**dass** die Gelenkpfanne aus einem Polymer besteht.

3. Hüftgelenk-Endoprothese nach Anspruch 2,
**dadurch gekennzeichnet,**
**dass** die Gelenkpfanne aus vernetztem oder unvernetztem UHMWPE besteht.

4. Hüftgelenk-Endoprothese nach einem der Ansprüche 1 bis 3,
**dadurch gekennzeichnet,**
**dass** die Membran (10) zwischen einer knochenseitigen Ausmündung (12) und einer Schulter (11) der Bohrung (8) ausgebildet ist.

5. Hüftgelenk-Endoprothese nach Anspruch 4,
**dadurch gekennzeichnet,**
**dass** die Membran (10) kegelförmig geformt ist.

6. Hüftgelenk-Endoprothese nach Anspruch 5,
**dadurch gekennzeichnet,**
**dass** ein Öffnungswinkel (α) der kegelförmigen Membran (10) etwa 120° beträgt.

7. Hüftgelenk-Endoprothese nach Anspruch 5 oder 6,
**dadurch gekennzeichnet,**
**dass** die Membran (10) so orientiert ist, dass eine Spitze der kegelförmigen Membran (10) in einer Montagerichtung der zumindest einen in die zumindest eine Bohrung (8) einbringbare Schraube weist.

8. Hüftgelenk-Endoprothese nach einem der Ansprüche 1 bis 3,
**dadurch gekennzeichnet,**
**dass** die Membran (10) an einer knochenseitigen Ausmündung (12) der Bohrung (8) ausgebildet ist.

9. Hüftgelenk-Endoprothese nach Anspruch 8,
**dadurch gekennzeichnet,**
**dass** die Bohrung (8) durch die Membran (10) knochenseitig glatt abgeschlossen ist.

10. Hüftgelenk-Endoprothese nach einem der Ansprüche 1 bis 3,
**dadurch gekennzeichnet,**
**dass** die Membran (10) an einer gleitflächenseitigen Ausmündung (14) der Bohrung (8) ausgebildet ist.

11. Hüftgelenk-Endoprothese nach Anspruch 9,
**dadurch gekennzeichnet,**
**dass** die Bohrung (8) durch die Membran (10) gleitflächenseitig glatt abgeschlossen ist.

12. Hüftgelenk-Endoprothese nach Anspruch 9 oder 10,
**dadurch gekennzeichnet,**
**dass** die Position der zumindest einen Bohrung (8) durch eine Markierung (15) gekennzeichnet ist.

13. Hüftgelenk-Endoprothese nach Anspruch 12,
**dadurch gekennzeichnet,**
**dass** die Markierung (15) ein Überstand oder eine Farbmarkierung ist.

## Claims

1. Hip joint endoprosthesis (1) which on a femoral component (3) has a j oint ball (7) which forms a sliding pairing with a one-piece joint socket (2) made of plastic,
wherein the joint socket (2) can be anchored in a pelvic bone with at least one screw and the at least one screw can be placed in at least one bore (8) and wherein the at least one bore (8) is closed by a barrier, wherein the barrier is a membrane (10),
wherein the membrane (10) is present in one piece with the joint socket (2), and
wherein the membrane (10) is embodied in such a way that the screw pierces the membrane (10) when it is inserted into the bore (8).

2. Hip joint endoprosthesis according to claim 1,
**characterised in that** the joint socket is made of a polymer.

3. Hip joint endoprosthesis according to claim 2,
**characterised in that** the joint socket is made of crosslinked or uncrosslinked UHMWPE.

4. Hip joint endoprosthesis according to one of claims 1 to 3,
**characterised in that** the membrane (10) is formed between a bone side opening (12) and a shoulder (11) of the bore (8).

5. Hip joint endoprosthesis according to claim 4,
**characterised in that** the membrane (10) is cone-shaped.

6. Hip joint endoprosthesis according to claim 5,
**characterised in that** an aperture angle (α) of the cone-shaped membrane (10) is approximately 120°.

7. Hip joint endoprosthesis according to claim 5 or 6,
**characterised in that** the membrane (10) is oriented so that an apex of the cone-shaped membrane (10) points in a mounting direction of the at least one screw which can be inserted into the at least one bore (8).

8. Hip joint endoprosthesis according to one of claims 1 to 3,
**characterised in that** the membrane (10) is formed in a bone side opening (12) of the bore (8).

9. Hip joint endoprosthesis according to claim 8,
**characterised in that** the bore (8) is closed by the membrane (10) on the bone side so that it is smooth.

10. Hip joint endoprosthesis according to one of claims 1 to 3,
**characterised in that** the membrane (10) is formed in a sliding face side opening (14) of the bore (8).

11. Hip joint endoprosthesis according to claim 9,
**characterised in that** the bore (8) is closed by the membrane (10) on the sliding face side so that it is smooth.

12. Hip joint endoprosthesis according to claim 9 or 10,
**characterised in that** the position of the at least one bore (8) is marked by a marking (15).

13. Hip joint endoprosthesis according to claim 12,
**characterised in that** the marking (15) is a raised portion or a coloured marking.

## Revendications

1. Endoprothèse de l'articulation de la hanche (1), qui comporte au niveau d'un composant fémur (3) une bille d'articulation (7) qui forme un appariement de glissement avec une cupule d'articulation (2) d'une pièce en matière synthétique,
où la cupule d'articulation (2) peut être ancrée avec au moins une vis dans un os du bassin et la au moins une vis peut être placée dans au moins un perçage (8) et où le au moins un perçage (8) est fermé par une barrière, où la barrière est une membrane (10), où la membrane (10) est d'une pièce avec la cupule d'articulation (2), et
où la membrane (10) est formée de telle manière que la vis traverse la membrane (10) lors de l'introduction dans le perçage (8).

2. Endoprothèse de l'articulation de la hanche selon la revendication 1, **caractérisée en ce que** la cupule d'articulation consiste en un polymère.

3. Endoprothèse de l'articulation de la hanche selon la revendication 2, **caractérisée en ce que** la cupule d'articulation consiste en UHMWPE réticulé ou non réticulé.

4. Endoprothèse de l'articulation de la hanche selon l'une des revendications 1 à 3, **caractérisée en ce que** la membrane (10) est formée entre une ouverture de sortie côté os (12) et un épaulement (11) du perçage (8).

5. Endoprothèse de l'articulation de la hanche selon la revendication 4, **caractérisée en ce que** la membrane (10) est formée de manière conique.

6. Endoprothèse de l'articulation de la hanche selon la revendication 5, **caractérisée en ce qu'**un angle d'ouverture (α) de la membrane conique (10) est d'environ 120°.

7. Endoprothèse de l'articulation de la hanche selon la revendication 5 ou 6, **caractérisée en ce que** la membrane (10) est orientée de telle manière qu'une pointe de la membrane conique (10) est tournée dans une direction de montage de la au moins une vis qui peut être introduite dans le au moins un perçage (8).

8. Endoprothèse de l'articulation de la hanche selon l'une des revendications 1 à 3, **caractérisée en ce que** la membrane (10) est formée au niveau d'une ouverture de sortie côté os (12) du perçage (8).

9. Endoprothèse de l'articulation de la hanche selon la revendication 8, **caractérisée en ce que** le perçage (8) est fermé par la membrane (10) de manière lisse côté os.

10. Endoprothèse de l'articulation de la hanche selon l'une des revendications 1 à 3, **caractérisée en ce que** la membrane (10) est formée au niveau d'une ouverture de sortie côté surface de glissement (14) du perçage (8).

11. Endoprothèse de l'articulation de la hanche selon la revendication 9, **caractérisée en ce que** le perçage (8) est fermé par la membrane (10) de manière lisse côté surface de glissement.

12. Endoprothèse de l'articulation de la hanche selon la revendication 9 ou 10, **caractérisée en ce que** la position du au moins un perçage (8) est **caractérisée par** un marquage (15).

13. Endoprothèse de l'articulation de la hanche selon la revendication 12, **caractérisée en ce que** le marquage (15) est un relief ou un marquage coloré.
